# EUROPEAN PATENT APPLICATION

(11) **EP 3 120 700 A1**
(43) Date of publication of application: **25.01.2017**
(21) Application number: 15002197.0
(22) Date of filing: 24.07.2015
(51) Int. Cl.: A01K 67/027, C12N 15/877

(54) **MICROINJECTION INTO A CELL'S NUCLEUS FOLLOWING SOMATIC CELL NUCLEAR TRANSFER**

(71) Applicant: Ludwig-Maximilians-Universität München, 80539 München (DE)
(72) Inventor: Kurome, Mayuko, 85764 Oberschleißheim (DE); Wolf, Eckhard, 85256 Vierkirchen (DE); Leuchs, Simon, 80689 München (DE)
(74) Representative: Müller-Boré & Partner Patentanwälte PartG mbB

(57) **Abstract**

The present invention relates to methods for the generation of a genetically modified animal, said methods comprising the steps of providing an oocyte of the animal that has undergone somatic cell nuclear transfer, and introducing at least one agent into a pronucleus-like structure by microinjection after said fused oocyte has developed said pronucleus-like structure, wherein said agent is selected from the group consisting of a transgene, a nucleic acid having homology to a genomic region of interest in the animal's genome, a protein, and mixtures thereof. The present invention further relates to respective genetically modified animals and progeny thereof.

SEQUENCE LISTING

<110>  LMU München

<120>  Microinjection into a cell's nucleus following somatic cell
       nuclear transfer

<130>  L 2213EU - kl

<160>  2

<170>  PatentIn version 3.5

<210>  1
<211>  19
<212>  DNA
<213>  Artificial Sequence

<220>
<223>  Primer

<400>  1
cgccatgaag atcgagtgc                                                    19


<210>  2
<211>  20
<212>  DNA
<213>  Artificial sequence

<220>
<223>  Primer

<400>  2
ccgaagtggt agaagccgta                                                   20

## Description

The present invention relates to methods for the generation of a genetically modified animal, said methods comprising the steps of providing an oocyte of the animal that has undergone somatic cell nuclear transfer, and introducing at least one agent into a pronucleus-like structure by microinjection after said fused oocyte has developed said pronucleus-like structure, wherein said agent is selected from the group consisting of a transgene, a nucleic acid having homology to a genomic region of interest in the animal's genome, a protein, and mixtures thereof. The present invention further relates to respective genetically modified animals and progeny thereof.

Genetic modification of animals is an established and feasible approach for pharmaceutical research. In particular, specific models for human diseases can be generated by targeted inactivation or introduction of genes as well as site directed mutagenesis.

A variety of methods to achieve this are known in the art. The key prerequisites for these methods are (i) having access to cells or tissue that can be manipulated and (ii) methods to introduce the change. The first of said prerequisites usually comprises access to primary cells, stem cells or induced pluripotent stem cells. For the latter of said prerequisites, most commonly used are transfections of DNA or RNA into cells by electroporation or by liposomes or nanocarriers. Alternatively, proteins can be introduced into cells to cause an effect, e.g. nucleases or recombinases.

The genetic modification of animals is established and more readily applicable if embryonal or induced pluripotent stem cells are available. This, however, is only available in mice, rats, hamsters, rabbits or monkeys, but not for other scientifically relevant animals. Especially large animals like pigs or cattle cannot yet be genetically modified as efficiently as the former. However, they continuously gain importance in therapeutic research.

The following methods are currently available to achieve genetic modifications that can be used independent from stem cells using somatic cells.

Somatic primary cells can be modified with the methods mentioned above. However, they are genomically differentiated further than stem cells and only have a shorter time available under culture conditions due to *in vitro* senescence. Homologous recombination of related sequences into the genome as well as random integration of exogenous gene sequences is also less efficient.

The exogeneous DNA can be introduced into somatic cells by the above-mentioned methods of electroporation, lipofection or automated microinjection. The cells modified in this way then can be fused with enucleated oocytes with the somatic cell nuclear transfer (SCNT) technology. Afterwards, the fused oocytes can be artificially activated and implanted into surrogate mothers via embryo transfer.

However, these primary cells cannot be cultured indefinitely and thus limit the possibilities for genetic modifications. In particular, there is usually not enough culture time to introduce more than one or two modifications with the necessary screening steps.

Further, culture time severely limits the cells ability to undergo SCNT and embryo transfer (ET) and produce viable offspring. In such a case, the cells have to be "rejuvenated" to further modify the cells. This entails a SCNT and embryo transfer into a surrogate mother. The established pregnancy is aborted usually after 30 to 40 days but this varies between species. The fetuses are isolated from the uterus and new fetal primary cells are isolated. These cells can now be modified anew. However, this rejuvenation step cannot be repeated more than 4 to 7 times as the SCNT/ET efficiency drops significantly. Moreover, each rejuvenation step takes 4 to 6 weeks and contains a certain risk of unwanted pregnancy terminations.

Another method to introduce genetic modifications in this context is the modification in oocytes in contrast to somatic cells. This type of modification is usually based on microinjection of DNA, RNA or protein into a zygote or oocyte.

Typically this entails an injection into the cytoplasma, more commonly into the male pronucleus of the cell after fertilization. It can also be achieved by sperm absorbed DNA and intracytoplasmic sperm injection (ICSI)-mediated gene transfer.

One advantage of these methods, and one reason why they are still performed in the field, is the possibility to introduce large DNA constructs like BACs or plasmids larger than 20 to 30 kilobases into cells and thus the genome. These large constructs are typically fragile to handle and are known to break down e.g. by mechanical stress or the harsh conditions of electroporation or similar methods. Microinjection as a method is more gentle on the DNA as smaller purely mechanical forces act on the DNA.

The introduction of larger DNA molecules can be a necessity for some genetic modifications. This is especially the case when genes should be introduced in total, including most of their natural regulatory and promoter sequences as well as downstream signals like polyA and transcription stops. Also, the innate exon-intron structure of a gene can be important e.g. to reach physiological expression of the gene, but at the same time elongates the DNA molecule needed. Introducing the whole genomic locus of the transgene is also thought to mitigate potential gene silencing by introduction site-specific effects.

An advantage of pronuclear injection or ICSI mediated gene transfer is the relative flexibility of the genome at the early developmental stage to take up foreign DNA. The resulting animals also carry a diverse gene pool, as they are derived from two germ cells. This broad gene pool is a requisite for the breeding and expanding of an animal herd especially for large animals where inbred lines are not available.

The above germ cell modification methods however come with some limitations. The first is the availability and the isolation of mature enough oozytes or zygotes to perform the modifications. For some species like pigs, this limitation can be overcome by superovulation and, for zygotes and pronuclear injection, the subsequent *in vitro* fertilization. This problem is intensified to the point of eliminating the possibility of genetic modification by these methods altogether in cases where the oocytes/zygotes cannot be isolated. This can be the case for example in some transgenic animals which never reach sexual maturity due to their genotype. In these cases, there were experiments to artificially mature immature germ cells for example by transplanting them into the mice for further xeno-in *vivo* maturation. However, in cases of some mutations that e.g. render the animals in question immune-incompetent this option might not be feasible. This is especially the case for larger animals that cannot be kept under SPF (specific pathogen free) conditions within reasonable effort. Also, when the genetically modified animal would carry an embryonic lethal genotype, these means of genetic modifications fall out of the scientists' toolbox.

To generate IVF (*in vitro* fertilization) zygotes, the conductor of the experiment also needs male germ cells to perform the fertilization. This poses the problem of availability of mature male germ cells that also have to be kept stored and handled. For cattle, these processes are well established and cryoconserved sperm is obtained and handled routinely by experts in the field. For other large animals like for example the pig there are still severe limitations in storage and handling.

Some transgenic or even naturally occurring genotypes in animals do not allow the derivation of germ cells due to an early lethal phenotype. In this case, only somatic cells are available from the fetus or the newborn. This limits the research and the potential rescue of problematic genotypes especially in genotypes that can only be rescued by large constructs that rely on the ICSI-mediated gene transfer (ICSI-MGT) or classic pronuclear microinjection.

Accordingly, the technical problem underlying the present invention is to provide methods for the genetic modification of animals circumventing the above limitations of methods known in the art. In particular, the methods of the present invention should be easy and practicable and combine the advantages of ICSI or pronuclear microinjection with the flexibility of somatic cell nuclear transfer.

The solution to the above technical problem is achieved by the embodiments characterized in the claims.

In particular, in a first aspect, the present invention relates to a method for the generation of a genetically modified animal, said method comprising the steps of:
(a) providing an oocyte of the animal that has undergone somatic cell nuclear transfer; and
(b) after said oocyte has developed a pronucleus-like structure (pseudo-pronucleus), introducing at least one agent into said pronucleus-like structure by microinjection, wherein said agent is selected from the group consisting of a transgene, a nucleic acid having homology to a genomic region of interest in the animal's genome, a protein, and mixtures thereof.

The term "genetically modified animal" as used herein relates to animals that have undergone any modification of their native genome. This includes transgenic animals, *i.e*., animals that have been genetically modified by introduction of a transgene into the animals' genome. Further, this includes animals whose native genome has been modified by homologous recombination-directed and/or nuclease-directed genome editing.

In a particularly preferred embodiment, the animal is a non-human animal, preferably a non-human mammal. Preferably, the animal is selected from the group consisting of mice, rats, hamsters, monkeys, rabbits, sheep, pigs, horses, dogs, cats, and cattle.

As used herein, the term "oocyte of the animal" indicates the fact that the oocyte is derived from any animal of the same species. However, said oocyte has not to be derived from the same individual as the somatic cell used for somatic cell nuclear transfer (SCNT).

The term "oocyte that has undergone somatic cell nuclear transfer" as used herein relates to an oocyte that has been subjected to SCNT as known in the art. It includes any oocytes that have been enucleated and into which the genetic material of a somatic cell has been introduced.

Methods for SCNT are not particularly limited and are known in the art. They include e.g. methods as described in the Examples hereinafter.

In a particular embodiment, providing an oocyte of the animal that has undergone somatic cell nuclear transfer in step (a) of the method of the present invention comprises the steps of:
(a1) providing a somatic cell of the animal;
(a2) providing an oocyte of the animal;
(a3) removing the nucleus from the oocyte provided in step (a2), resulting in an enucleated oocyte; and
(a4) fusing said somatic cell with the enucleated oocyte obtained in step (a3).

In this context, the term "somatic cell of the animal" as used herein relates to any somatic cell, *i.e*., any cell other than a gamete, germ cell, gametocyte or undifferentiated stem cell. Said cell is derived from any animal of the same species, but does not have to be derived from the same individual as the oocyte used for somatic cell nuclear transfer (SCNT). Preferably, the somatic cell is a primary somatic cell, *i.e*., a somatic cell that is directly taken from living tissue.

Means for removing the nucleus from an oocyte and means for fusing a somatic cell with an enucleated oocyte are not particularly limited and are known in the art. The latter include electro-fusion methods as known in the art, e.g. as described in the Examples hereinafter.

In another particular embodiment, providing an oocyte of the animal that has undergone somatic cell nuclear transfer in step (a) of the method of the present invention comprises the steps of:
(a1) providing a somatic cell of the animal;
(a2) providing an oocyte of the animal;
(a3) isolating the nucleus from the somatic cell provided in step (a1);
(a4) removing the nucleus from the oocyte provided in step (a2), resulting in an enucleated oocyte; and
(a5) introducing the isolated somatic cell nucleus obtained in step (a3) into the enucleated oocyte obtained in step (a4).

Means for isolating the nucleus from a somatic cell and means for introducing an isolated somatic cell nucleus into an enucleated oocyte are not particularly limited and are known in the art. The latter include intracytoplasmic injection (ICI) methods as known in the art.

Oocytes that have undergone SCNT typically develop a pronucleus-like structure (pseudo-pronucleus). Development of this structure is typically seen at 8 to 10 hours after SCNT. However, not all oocytes that have undergone SCNT do develop a pronucleus-like structure. According to the present invention, only oocytes that do develop a pronucleus-like structure are used. The pronucleus-like structure is a diploid nucleus that is visually comparable in size to the regular pronuclei found e.g. after IVF or ICSI.

According to the present invention, the at least one agent introduced into the pronucleus-like structure in the method of the present invention is selected from the group consisting of a transgene, a nucleic acid having homology to a genomic region of interest in the animal's genome, a protein, and mixtures thereof.

The transgene that may be introduced into the pronucleus-like structure of the oocyte according to the method of the present invention is not particularly limited and includes any form of nucleic acid that (i) contains at least a coding sequence for a protein or functional RNA of interest and/or a regulatory genetic element, and (ii) can integrate into the animal genome. Preferably, the transgene is an RNA or DNA molecule, wherein DNA molecules, in particular linear DNA molecules are preferred. In particular embodiments, the transgene includes at least one gene encoding a particular protein or functional RNA of interest, optionally including the native exon-intron structure, and further optionally including regulatory sequences, promoter sequences, and downstream signals such as poly-adenylation (polyA) signals or transcription stop signals. Respective sequences and signals are not particularly limited and are known in the art. In a particular embodiment, the transgene includes the complete genomic locus of at least one particular gene of interest. In another specific embodiment, the transgene includes a bacterial artificial chromosome (BAC). In certain embodiments, the transgene is at least 20 kilobases (kb), at least 30 kb or at least 50 kb in length.

The nucleic acid having homology to a genomic region of interest in the animal's genome that may be introduced into the pronucleus-like structure of the oocyte according to the method of the present invention is not particularly limited and includes any form of nucleic acid that (i) shows homology (sequence identity) to said genomic region, *i.e*., that is capable of binding to a DNA strand in said genomic region by Watson-Crick pairing allowing for a crossing over event, and (ii) is capable of acting as a template for homologous recombination-directed gene editing and/or acting as guiding nucleic acid for nucleases such as e.g. Cas9 (CRISPR associated protein 9). Thus, in a preferred embodiment, the nucleic acid having homology to a genomic region of interest in the animal's genome comprises a target sequence for a nuclease, e.g. CRISPRs (clustered regularly interspaced short palindromic repeats), and/or a target sequence for a recombinase, e.g. a loxP or attB site. Preferably, the nucleic acid is an RNA or DNA molecule, wherein DNA molecules, in particular linear DNA molecules are preferred. Respective nucleic acids are known in the art.

The protein that may be introduced into the pronucleus-like structure of the oocyte according to the method of the present invention is not particularly limited and includes nucleases, such as e.g. Cas9, Transcription Activator-like Effector Nucleases (TALENs), Zinc Finger Nucleases, Fokl nuclease, and other Meganucleases, as well as recombinases, such as e.g. Cre recombinase, Flp recombinase, and PhiC31 recombinase.

According to the present invention, at least one, *i.e*., one, two, three, or more, agent may be introduced into the pronucleus-like structure of the oocyte. This includes e.g. combinations/mixtures of nucleic acids and proteins, such as e.g. Cas9 and CRISPRs.

Methods for microinjection into cells or cellular structures such as a pronucleus-like structure are not particularly limited and are known in the art.

The method of the present invention is performed *in vitro, i.e.,* it is not performed on the human or animal body.

In certain embodiments, the method of the present invention further comprises the step of
(c) activating the genetically modified oocyte obtained in step (b).

In this context, means for activating an oocyte are not particularly limited and are known in the art. They include electrical activation as known in the art, e.g. as described in the Examples hereinafter.

In certain embodiments, the method of the present invention further comprises the step of
(d) implantation of the activated oocyte or of the embryo developed from said activated oocyte into a surrogate mother by embryo transfer.

Suitable methods for embryo transfer into suitable surrogate mothers are not particularly limited and are known in the art. An exemplary method is described in the Examples hereinafter.

In a further aspect, the present invention relates to a genetically modified animal produced by a method of the present invention. In a yet further aspect, the present invention relates to progeny of said genetically modified animal.

As used herein, the terms "comprises/comprising", "consists/consisting of", and "consists/consisting essentially of" are used interchangeably, *i.e.,* any of said terms can be exchanged against any of the other two.

The methods of the present invention only rely on somatic cells of the animal that is to be modified. The enucleated oocytes can be derived from any animal of the species. After fusion of the somatic cell and the oocyte, the already diploid nucleus enlarges into a pronucleus-like structure (pseudo-pronucleus), comparable in size to the pronuclei found e.g. after IVF or ICSI. This already diploid nucleus does not have to undergo as big rearrangements as male and female pronuclei merging. This means it can be modified with currently available microinjection technology. Also, the handling of oocytes can be done according to standard procedures, thus making these methods easy and practicable to insert into current workflows in the laboratories.

Thus, the methods of the present invention incorporate the advantages of ICSI or pronuclear microinjection into the flexibility of somatic cell nuclear transfer. Accordingly, for example genotypes that would be lethal before sexual maturity can still be addressed with microinjection technology.

Said methods further allow the introduction of DNA in a second step after *in vitro* primary cell culture. Here, up to now cells sometimes reached their senescence state before all necessary genetic modifications could be introduced. Now, with the methods of the present invention, the necessary rejuvenation step of primary cells through a somatic cell nuclear transfer / embryo transfer / generating rejuvenated fetal primary cells can be used for another DNA introduction.

Thus said methods save time of up to several months and also reduce the number of animals needed for the embryo transfers and aborted pregnancies to get to fetal material.

This new way to introduce genetic modification adds to the tool base of molecular biologists, geneticists etc. It facilitates the establishment e.g. of genetic models for human diseases such as diabetes or cancer. These model animals are important cornerstones of modern therapeutic research. Better or novel ways to genetic modification speed up these developments, thus saving lives of lesser suited animal models by generating more relevant preclinical data. Such tools allow more complex genetic modifications, ideally mimicking more closely the human phenotype/pathology. It can also be used as a tool to correct genetic defects in cells and thus in cloned animals. Monogenetic diseases in animals could be corrected with this method.

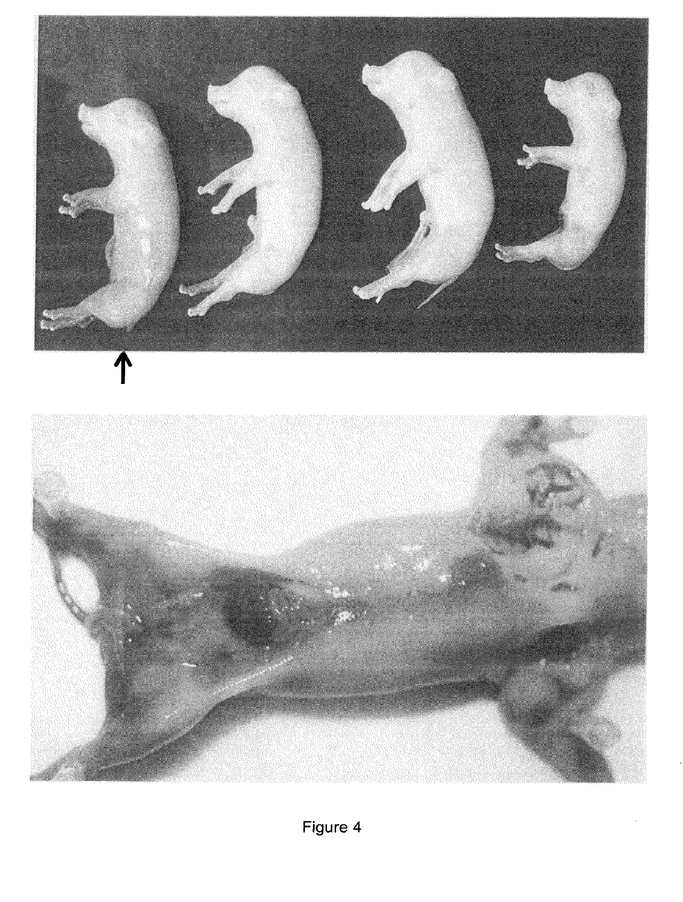

The figures show:
Figure 1:
   Remodeling pattern of a donor nucleus at different points in time after nuclear transfer. Nuclear structure is shown 1 h (a, b), 3 h (c, d), and 8 h (e, f) after somatic cell nuclear transfer. The structure forming at 8 h is a pronucleus-like structure (pseudo-pronucleus) suitable for microinjection. Said pronucleus-like structure shows a similar structure to common male of female pronuclei.
Figure 2:
   Schematic (upper panel) and microscopic (lower panel) depiction of the method of the present invention, showing microinjection of a transgene into the pronucleus-like structure (black arrow) of a nuclear transfer (NT) embryo. The pronucleus-like structure can be visualized in cloned pig embryos.
Figure 3:
   GFP-expressing blastocysts produced by the method of the present invention. A-C: non-treatment group; D-F: DNA injected group; A, D: brightfield; B, E: fluorescence; C, F: fluorescence and brightfield.
Figure 4:
   Embryos produced by the method of the present invention show *in vivo* developmental ability. Four fetuses (1-4) were collected at day 68 (upper panel), of which one (red arrow) showed laparoschisis (lower panel).
Figure 5:
   Green fluorescence was visible in the extremities of one fetus (fetus 3) of the fetuses shown in Figure 4, indicating that the transgene is expressed *in vivo.*
Figure 6:
   PCR was performed using GFP-specific primers on different organs and tissues of the four fetuses shown in Figure 4 (fetus 1: upper left panel; fetus 2: upper right panel; fetus 3: lower left panel; fetus 4: lower right panel). The transgene was introduced into all major organs and the placenta in fetus 3. (1: Liver, 2: Pancreas, 3: Heart, 4: Lung, 5: Kidney, 6: Muscle, 7: Placenta, 8: skin leg, 9: skin back 10: skin ear, p: positive control, w: wildtype DNA negative control, n: water negative control, M = DNA marker).
Figure 7:
   Single cell colonies from kidney cells of fetus 3 show GFP expression *in vitro. 84* single cell clones could be screened for GFP expression by PCR. Of these, 36 clones (43%) showed non-integration and non-expression in culture, 43 clones (51%) showed integration but non-expression in culture, and 5 clones (6%) showed integration and expression in culture. Kidney cells isolated from fetus 3 are shown (a: brightfield; b: fluorescence; c: merge).

The present invention will be further illustrated in the following examples without being limited thereto.

### Examples

### Experimental procedures:

### DNA Preparation for injection.

A plasmid expressing pmaxGFP under a CMV promoter was linearized by restriction digest and precipitated with phenol chloroform. The DNA was resolved in TE buffer for microinjection and diluted to a concentration of 10ng/µl.

### Somatic Cell Nuclear Transfer (SCNT).

SCNT was performed using *in vitro* matured (IVM) oocytes as recipient cytoplasts as known in the art. Briefly, cumulus-oocyte complexes (COCs) were collected by aspiration from antral follicles (3.0-6.0mm in diameter) on the surface of ovaries obtained from a local abattoir. COCs displaying > 3 layers of compact cumulus cells were selected and cultured in NCSU based maturation medium for 40-42 h. For the first 22 h, COCs were cultured in maturation medium with hormones, and then for the following 18-20 h without hormones in a humidified atmosphere of 5% CO₂, 5% O₂, and 90% N₂ at 38.5 °C. After maturation, oocytes with expanded cumulus cells were treated with hyaluronidase (1 mg/ml), and cumulus cells were removed from oocytes by gentle pipetting. Only oocytes showing evenly granulated ooplasm and extrusion of the first polar body were selected as matured oocytes and used for the experiments.
The matured oocytes were cultured in a NCSU23 medium supplemented with 0.4 µg/ml demecolcine, 0.05 M sucrose and 4 mg/ml BSA for 0.5-1 h and then enucleated by aspirating the membrane protrusion, the first polar body and adjacent cytoplasm using a beveled pipette (30 µm in diameter). In the case of *in vitro* experiments, the successful removal of the chromosomes was checked by staining the cytoplasts with 5 µg/ml bisbenzimide (Hoechst 33342).
Primary kidney cells from a 3 months old piglet were used as nuclear donors after cell cycle synchronization by serum starvation for 48 h. A single donor cell was inserted into the perivitelline space of an enucleated oocyte. Membrane fusion was induced using an electric cell fusion generator and two electrode needles attached to micromanipulators by applying a single direct current (DC) pulse (200 V/mm, 20 µs ×1) and a pre- and post-pulse alternating current (AC) field of 5 V, 1 MHz for 5 s, respectively, to each single oocyte/donor cell complex in fusion medium (Eppendorf, Hamburg, Germany). After 0.5-1 h culture in NCSU23, SCNT embryos were placed in an activation solution consisting of 0.3 M mannitol, 50 µM CaCl2, 100 µM MgSO4, and 0.01% PVA and activated by a single DC pulse of 150 V/mm for 100 µs. Activated oocytes were treated with 5 µg/ml CB for 3 h, then transferred into a Porcine Zygote medium-5 (PZM-5; Functional Peptide, Co., Yamagata, Japan) until the following experiments.

### In vitro produced zygotes.

*In vitro* fertilization (IVF) of *in vitro* matured oocytes was performed using same batch of frozen sperm derived from commercially available bore semen (Duroc). Briefly, frozen-thawed semen was washed three times with Beltsville thawing solution (BTS; 1,000 x g, 4 min), and was re-suspended in porcine fertilization medium (PFM; Research Institute for the Functional Peptides, Yamagata, Japan) at a concentration of 1 × 10⁷ cells/ml. For insemination, around 20 oocytes with expanded cumulus cells were co-incubated with spermatozoa (1.0 × 10⁶ cells/ml) in a 100 µl drop of PFM for 7 h in a humidified atmosphere of 5% CO₂, 5% O₂, and 90% N₂ at 38.5 °C. After insemination, cumulus cells and excess sperm were removed from the eggs, and only eggs observed two polar bodies were used for the experiments.

### Introduction of the transgene into a pronucleus-like structure of the cloned embryos.

For visualization of a pronucleus-like structure, centrifugation was done 10 h after nuclear transfer (10.000 × g, 38 °C, 20 min in TL-HEPES-PVA). SCNT embryos with the visible structure were selected and directly injected the linearized plasmid expressing pmaxGFP under a CMV promoter (10ng/µl; AMAXA Biosystems) into the PN-like structure of the cloned embryos. As a control, the classical pronuclear injection was performed using *in vitro* produced zygotes. DNA injected embryos were cultured in PZM-5 for 7 days to evaluate GFP expression in the blastocysts under a fluorescence microscope (488/530 nm) or cultured until embryo transfer.

### Embryo Transfer.

Embryo transfer was carried out with laparoscopy. DNA injected embryos showing normal development were transferred into the recipients without selection of GFP positive. Pregnancy was monitored by ultrasound scanning at day 21, and the pregnant recipients were euthanized on Day 68 to collect fetuses.

### Isolation of DNA from fetal tissue.

The nexttec™ 1^{-Step} Tissue & Cells Kit was used to isolate DNA from fetal tissue according to manufacturer's instructions. 2µl of the DNA were used for PCR analysis.

### PCR confirmation of DNA integration.

The copGFP sequence was amplified using the primers GFP F (CGCCATGAAGATCGAGTGC; SEQ ID NO: 1) and GFP R (CCGAAGTGGTAGAAGCCGTA; SEQ ID NO: 2).

### Isolation of fetal primary cells.

Fetal kidney was minced mechanically with a scalpel and washed twice in PBS, minced and washed with DMEM by centrifugation (5-10 min; 180xg) until the supernatant became clear. Subsequently, the pelleted tissue pieces were resuspended in 15 ml Hank's Buffered Salt Solution (HBSS; PAA) with 0.1 % (w/v) collagenase II (Invitrogen) and incubated at 37°C while stirring for 1 to 1.5 h until dissolved. After incubation, flasks were filled up to 50 ml with DMEM, filtered through a 100-µm mesh and washed with DMEM (5-10 min, 180xg) until the supernatant became clear. Depending on pellet size 1/6 to 1/24 of the resuspended PKCs were seeded per 100 mm plate and all resuspended PEFs onto a 60 mm plate.

### Single cell cloning and cell culture.

Cells were cultured at 100% humidity and 37°C on collagen type 1-coated (Serva Electrophoresis, Heidelberg, Germany) cell culture dishes in medium containing Dulbecco's modified Eagle medium (DMEM; Invitrogen, Darmstadt, Germany) supplemented with 293 mg/l L-glutamine, 100 units/ml penicillin,100 µg/ml streptomycin (PAA, Pasching, Austria), 0.1 mM 2-mercaptoethanol (Sigma-Aldrich, Steinheim, Germany), 1% (v/v) nonessential amino acid and 1% (v/v) sodium pyruvate (Invitrogen) and 10% fetal calf serum (FCS; Invitrogen). Single cell cloning is achieved by dilution of cells onto collagen coated 96 well cell culture plates.

### Example:

A reporter plasmid carrying the copGFP fluorescent protein under the control of the strong and ubiquitous cytomegalovirus promoter (CMV) was chosen for the DNA injection experiments. Thus, a successful introduction and expression of the transgene can easily be visualized with common state of the art fluorescence microscopy. The presence of the plasmid DNA in cells or tissues can also be shown by PCR amplification of a part of the copGFP gene.

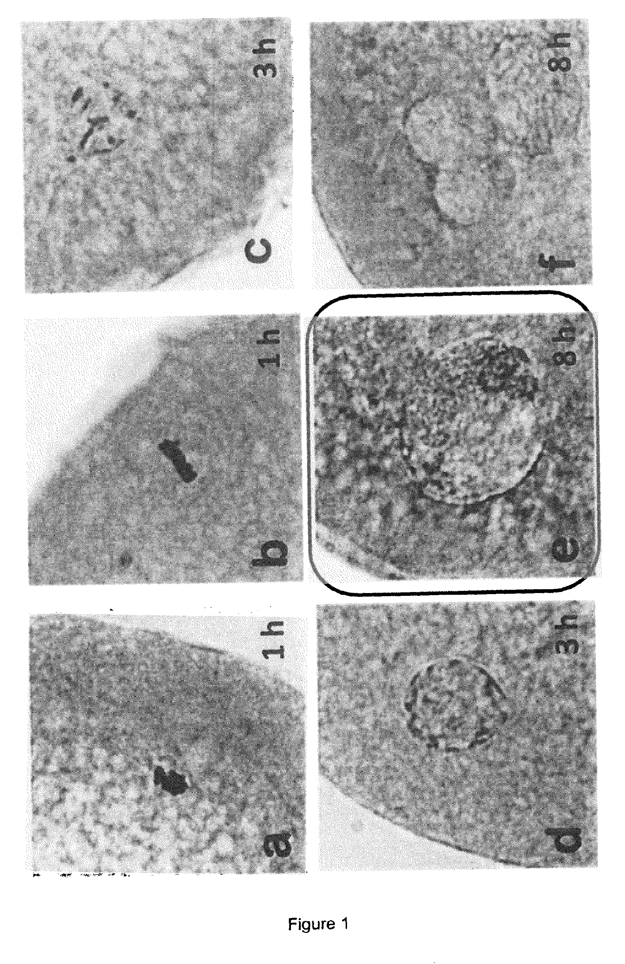

The idea of nuclear microinjection after somatic cell nuclear transfer (SCNT) was postulated when noting the similarity of the nuclear structure to the structure of *in vitro* fertilized oocyte nuclei (Fig. 1).

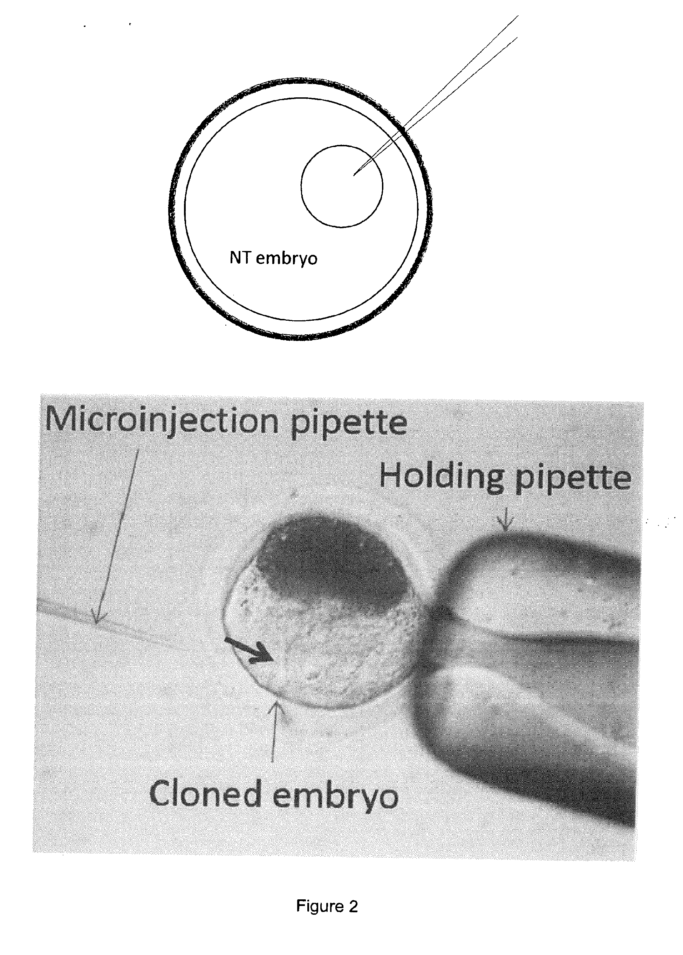

The nuclear structure could be visualized consistently in pig cells. Centrifugation was necessary to remove the lipids hindering visualization. The injection could be performed when the structure was visible. (Fig. 2).

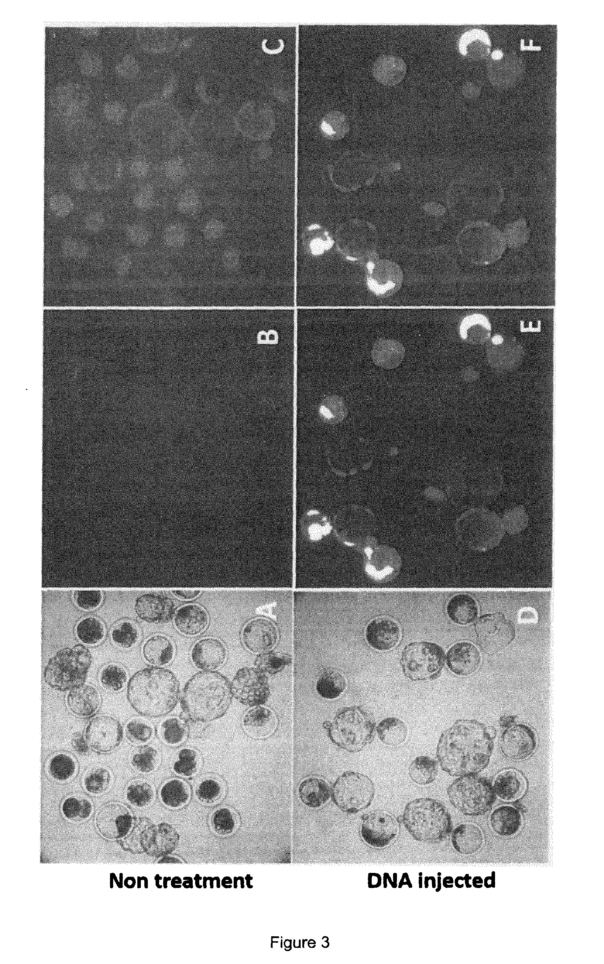

Next, the *in vitro* viability of the oocytes and their development to blastocysts was confirmed. There was a significant reduction in blastocyst development, but still a high enough percentage (22,4%) to continue to embryo transfer. The injection was performed with a linearized test plasmid expressing GFP in order to visualize successful injections. 57,7% of developing blastocysts showed GFP expression. (Table 1; and Fig. 3).

**Table 1: DNA injection influences early embryo development but still allows blastocyst formation and development.**

| **Groups** | **No. of embryos cultured** | **Normal cleaved embryos (%)** | **Embryos developed to blastocyst (%)** | **EGFP-positive blastocysts (%)** |
|---|---|---|---|---|
| non treatment | 68 | 49 (72.1) | **28 (41.2)^{a}** | 0 (0) |
| DNA injected | 116 | 62 (53.4) | **26 (22.4)^{b}** | **15/26 (57.7)** |
| | | | | **15/116 (12.9)** |

| | | | | |
|---|---|---|---|---|
| ^{a,b} Different letters within columns denote significant differences; four replicates were performed; nuclear transfer was performed with wildtype donor cells; pmaxGFP (3486 bp) was used as transgene (10ng/µl) | | | | |

Next, the nuclear microinjection was compared to the state of the art pronuclear microinjection and no significant differences were found. (Table 2).

**Table 2: Transgene expression efficiency in blastocysts generated by classical PN injection or nuclear injection is comparable.**

| **Method** | **No. of embryos cultured** | **Embryos developed to blastocyst (%)** | **GFP-positive blastocysts (%)^{c}** |
|---|---|---|---|
| Classical PN injection^{a} | 226 | 41 (18.1) | **68.4 (28/41)** |
| Nuclear injection^{b} | 307 | 40 (13.0) | **52.5 (21/40)** |

| | | | |
|---|---|---|---|
| Four replicates were performed; pmaxGFP (3486 bp) was used as transgene (10ng/µl); ^{a} GFP was injected into pronucleus of *in vitro* fertilized embryos; ^{b} GFP was injected into pronuclear-like structure of nuclear transfer (NT) embryos from wildtype cells; blastocysts were counted at day 7; ^{c} % of GFP-positive was counted out of total number of blastocysts | | | |

The next step after having shown the *in vitro* expression and developmental capability of the nuclear microinjection embryos was the generation of *in vivo* data. This is important, because the capability of the embryos to form a complete fetus could still be impaired after the handling procedure. However, the potential of the modified blastocysts to still form a complete fetus up to day 68 (of 114 days pregnancy in total) could be shown. This gives a good indication of the *in vivo* practicability of the method. The blastocysts were not preselected for green expression to also have control animals in the potential litter. Day 68 abortion offers sufficient information on developmental status as well as enough material for screenings without the threat of giving birth to potentially developmentally impaired piglets. (Table 3; and Figure 4).

**Table 3: Embryos produced by nuclear injection method show in vivo developmental ability.**

| **Recipient No.** | **Total No. of embryos transferred** | **Pregnancy** | **No. of fetuses on day 68** |
|---|---|---|---|
| 1 | 87 | + | - |
| 2 | 92 | - | - |
| 3 | 150 | + | **4** |
| 4 | 149 | + | - |

| | | | |
|---|---|---|---|
| Injected embryos (1-4 cell stage) were transferred into the recipients without selection of GFP positive; Pregnancy was checked at day 21, and fetuses were collected at day 68 | | | |

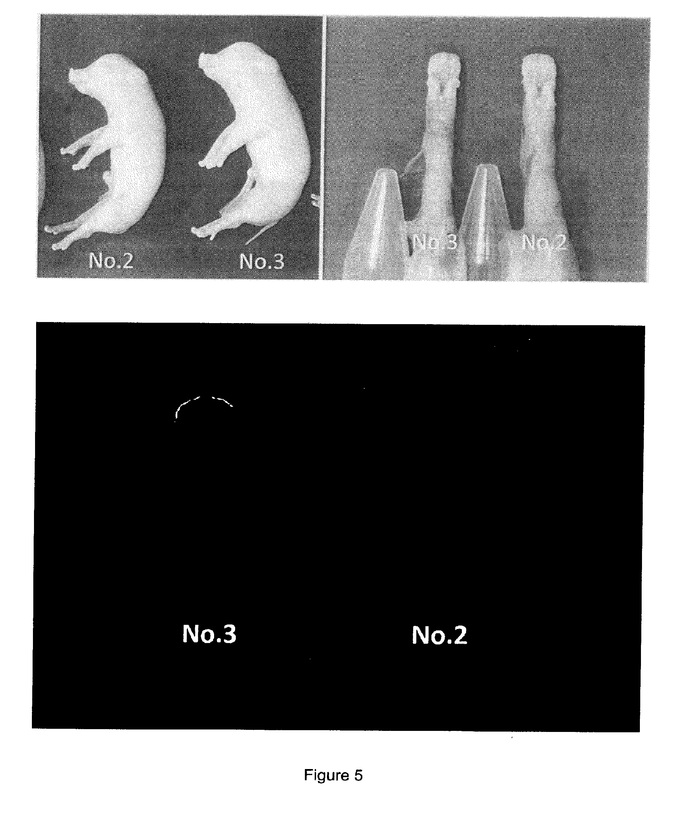

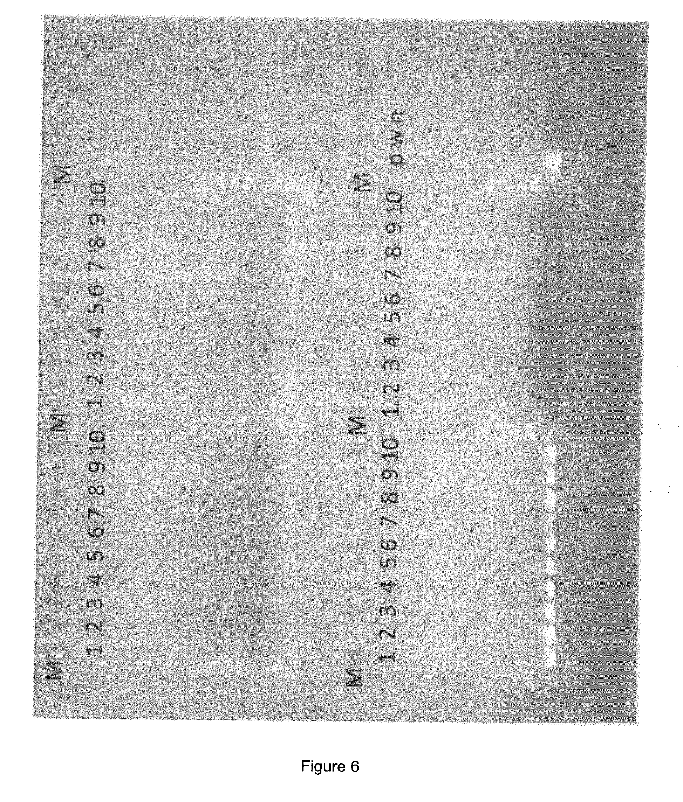

One of the four fetuses was indeed transgenic, as was observable from fluorescence imaging of the fetus' claws. (Fig. 5). Therefore, preparations of all fetuses of all organs were isolated and preserved. This allowed to test for chimerism of the integration. There was the potential of chimeric organisms with partial transgene integration, as the introduction of DNA happened already into a diploid nucleus. However, PCR analysis showed only the presence of the construct in all major organs of fetus 3 as well as its placenta. (Fig. 6).

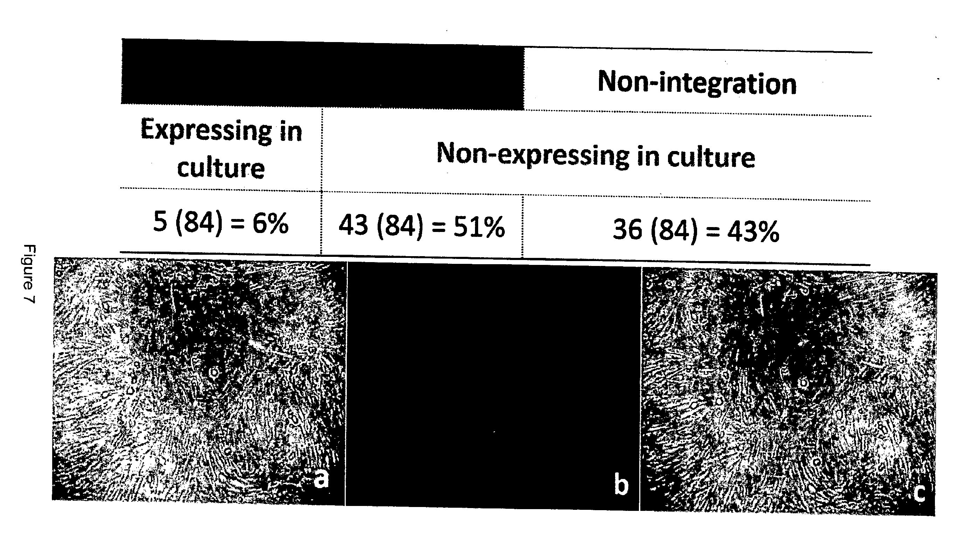

To confirm expression of the GFP at a cellular level and look for intra-organ mosaicism, single cell clones were isolated from the kidney of fetus 3. These clones were initially analyzed visually for fluorescence. This showed only a subset of clones expressing (Fig. 7). This is quite probably an artifact of the CMV promoter on the construct. Its viral origin, paired with positional effects during integration in the genome are known in some cases to silence the expression of transgenes. This is a "specialty" of the construct used. A more sophisticated choice of transgene, with positional insulators and endogenous promoters is better suited to avoid this problem. However this silencing does not mitigate the success of the method: In a second step, the integration of the construct into the genome was confirmed by PCR and could be detected in about half the cell clones (Figs. 6 and 7). This is another indicator of the silencing mentioned above. It also indicates a mosaic transgenic animal with mixed genotypes in its organs. This is a characteristic also found in animals created with the common pronucleus microinjection. It is a common problem, which cannot willingly be avoided but can easily be compensated for by increasing the number of transgenics generated.

## Claims

1. A method for the generation of a genetically modified animal, said method comprising the steps of:
(a) providing an oocyte of the animal that has undergone somatic cell nuclear transfer; and
(b) after said oocyte has developed a pronucleus-like structure (pseudo-pronucleus), introducing at least one agent into said pronucleus-like structure by microinjection, wherein said agent is selected from the group consisting of a transgene, a nucleic acid having homology to a genomic region of interest in the animal's genome, a protein, and mixtures thereof.

2. The method of claim 1, wherein providing an oocyte of the animal that has undergone somatic cell nuclear transfer in step (a) comprises the steps of:
(a1) providing a somatic cell of the animal;
(a2) providing an oocyte of the animal;
(a3) removing the nucleus from the oocyte provided in step (a2), resulting in an enucleated oocyte; and
(a4) fusing said somatic cell with the enucleated oocyte obtained in step (a3).

3. The method of claim 1, wherein providing an oocyte of the animal that has undergone somatic cell nuclear transfer in step (a) comprises the steps of:
(a1) providing a somatic cell of the animal;
(a2) providing an oocyte of the animal;
(a3) isolating the nucleus from the somatic cell provided in step (a1);
(a4) removing the nucleus from the oocyte provided in step (a2), resulting in an enucleated oocyte; and
(a5) introducing the isolated somatic cell nucleus obtained in step (a3) into the enucleated oocyte obtained in step (a4).

4. The method of any one of claims 1 to 3, wherein the animal is a non-human animal.

5. The method of claim 4, wherein the animal is selected from the group consisting of mice, rats, hamsters, monkeys, rabbits, sheep, pigs, horses, dogs, cats, and cattle.

6. The method of any one of claims 1 to 5, wherein the somatic cell is a primary somatic cell.

7. The method of any one of claims 1 to 6, wherein the transgene and/or the nucleic acid having homology to a genomic region of interest in the animal's genome is a DNA molecule.

8. The method of claim 7, wherein the DNA molecule is a linear DNA molecule.

9. The method of any one of claims 1 to 8, wherein the transgene is at least 20 kilobases (kb) in length.

10. The method of any one of claims 1 to 9, wherein the nucleic acid having homology to a genomic region of interest in the animal's genome comprises a target sequence for a nuclease and/or a target sequence for a recombinase.

11. The method of any one of claims 1 to 10, wherein the protein is selected from the group consisting of nucleases and recombinases.

12. The method of any one of claims 1 to 11, wherein said method is performed *in vitro.*

13. A genetically modified animal produced by a method of any one of claims 1 to 12.

14. Progeny of the genetically modified animal of claim 13.
